# FASCICULE DE BREVET EUROPEEN

(11) **EP 4 669 226 B1**
(45) Date de publication et mention de la délivrance du brevet: **18.11.2026**
(21) Numéro de dépôt: 24703797.1
(22) Date de dépôt: 08.02.2024
(51) Int. Cl.: A61B 17/30, B33Y 80/00, A61F 9/007, A61B 17/29, A61B 17/00

(54) **INSTRUMENT POUR CHIRURGIE OPHTALMIQUE**
INSTRUMENT FÜR DIE AUGENCHIRURGIE
INSTRUMENT FOR OPHTHALMIC SURGERY

(30) Priorité: 22.02.2023 FR 2301632
(43) Date de publication de la demande: 31.12.2025
(73) Titulaire: Moria, 03160 Bourbon-l'Archambault (FR)
(72) Inventeur: AUFAURE, Jean-Luc, 03160 Bourbon-l'Archambault (FR)
(74) Mandataire: Cabinet Boettcher
(86) Numéro de dépôt international: PCT/EP2024/053122
(87) Numéro de publication internationale: WO 2024/175367

(56) Documents cités:
- US-A1- 2014 128 909
- US-A1- 2014 142 603
- US-A1- 2019 357 928

## Description

La présente invention concerne le domaine des instruments utilisés dans la chirurgie ophtalmique.

La réalisation de chirurgies ophtalmiques et en particulier les chirurgies vitréo-rétiniennes nécessitent l'utilisation de nombreux instruments à usage unique ou pouvant être autoclavés, destinés à visualiser, découper, saisir, détacher les membranes qui se forment sur différentes parties de l'œil au cours de certaines pathologies.

Parmi ceux-ci, la pince-rétine à commande distale est spécifiquement utilisée pour saisir les membranes à éliminer. A l'état désactivée, la pince est ouverte, le praticien en actionnant la commande, en général par simple pression, va déclencher la fermeture de la pince. Dans la plupart des pinces-rétines existant à l'heure actuelle, la pince de la pince-rétine est insérée dans un tube, la partie active de la pince dépassant dudit tube. L'ouverture/fermeture (désactivation/activation) de la pince est réalisée par la translation du tube le long de la pince. Ainsi, pour fermer la pince, le praticien, va exercer une pression sur la commande qui va provoquer la translation du tube le long de la pince pour y engager la partie active de ladite pince et ainsi la fermer. La translation se fait dans une direction allant de l'extrémité proximale de l'instrument vers son extrémité distale opposée qui comprend la partie active de la pince. Pour ouvrir la pince, le praticien relâche la pression exercée sur la commande provoquant un mouvement inverse du tube pour dégager l'extrémité active de la pince (de l'extrémité distale vers l'extrémité proximale de l'instrument) et ainsi provoquer son ouverture.

Par exemple, une telle pince-rétine est décrite dans les demandes WO-A-2021/038428 ou WO-A-2018/156341 ou encore les demandes US2014/142603, US2014/128909 ou US 2019/357928.

Le nombre de pièces constitutives des pinces-rétines existantes est important et rend le processus de fabrication long et coûteux. En outre, l'agencement de ces différentes pièces rend le nettoyage difficile. Ainsi, pour éviter les contaminations liées à un mauvais nettoyage de l'instrument, les praticiens privilégient des pinces-rétines à usage unique. Il convient donc de concilier un prix de vente de la pince-rétine compatible avec un usage unique, donc relativement faible, alors même que les coûts de production liés au grand nombre de pièces constitutives et à la complexité du processus d'assemblage desdites pièces sont actuellement élevés. Les fabricants d'instruments chirurgicaux et notamment de pinces rétines ont par conséquent un souci constant de baisse des coûts de production sans pour autant sacrifier ni la qualité ni l'ergonomie des pinces-rétines.

### OBJET DE L'INVENTION

L'invention a notamment pour but de proposer un instrument de type pince-rétine dont la fabrication est simplifiée, moins onéreuse tout en préservant son ergonomie.

### RESUME DE L'INVENTION

A cet effet, on prévoit, selon l'invention un instrument pour la chirurgies ophtalmique comprenant :
- une pince avec une extrémité active et un fourreau recevant la pince à coulissement,
- un corps central avec une première extrémité proximale solidaire d'un manche et une deuxième extrémité distale à laquelle est fixée une première extrémité de la pince, ladite première extrémité étant opposée à l'extrémité active de la pince,
- un actionneur formé d'un ensemble de leviers s'étendant autour du corps central et ayant des premières extrémités reliées au corps central et des deuxièmes extrémités réunies par une douille à laquelle est fixé le fourreau, les leviers étant élastiquement déformables entre une première position désactivée de la pince dans laquelle l'extrémité active de la pince est ouverte et en saillie du fourreau et une deuxième position activée dans laquelle le fourreau vient exercer un effort sur l'extrémité active de la pince de manière à resserrer et forcer la fermeture de l'extrémité active de la pince. Selon l'invention, le corps central, l'actionneur et la douille de l'instrument sont une pièce monobloc.

Ainsi, l'instrument selon l'invention comprend un nombre très limité de pièces, faciles et économiques à produire et à assembler tout en conservant une excellente ergonomie d'utilisation pour le praticien.

L'instrument selon l'invention comprend en outre un nez de guidage recevant à coulissement au moins une partie du fourreau pour y être guidé, ledit nez de guidage est fixe et relié au corps central. De manière préférentielle, le nez de guidage est relié au corps central par des bras passant entre les leviers. Ledit nez de guidage peut comprendre en outre un logement recevant la douille à coulissement.

De manière avantageuse, le nez de guidage, le corps central, l'actionneur et la douille de l'instrument selon l'invention sont une pièce monobloc.

Le corps central et le manche de l'instrument peuvent en outre être d'une pièce monobloc.

L'invention concerne également un procédé de fabrication d'un instrument selon l'invention comprenant les étapes de
a. fabriquer en fabrication additive une pièce monobloc comprenant au moins, un corps central, un actionneur, une douille et un nez de guidage ;
b. fixer le fourreau dans la douille ;
c. insérer à coulissement la pince dans le fourreau et la fixer dans le corps central.

La fabrication additive permet d'obtenir rapidement et à moindre coût l'instrument selon l'invention.

D'autres caractéristiques et avantages de l'invention ressortiront à la lecture de la description qui suit d'un mode de réalisation particulier et non limitatif de l'invention.

### BREVE DESCRIPTION DES DESSINS

Il sera fait référence aux dessins annexés, parmi lesquels :
[Fig. 1] : vue légèrement en perspective d'un instrument selon l'invention, l'instrument étant désactivée, la pince en position ouverte ;
[Fig. 2] : vue en coupe longitudinale selon l'axe X d'un instrument selon l'invention, la pince étant en position ouverte et présentée à 90° par rapport à la figure 1.
[Fig. 3] : vue en coupe longitudinale selon l'axe X d'un instrument selon l'invention, la pince étant en position ouverte.
[Fig 4.] : vue agrandie selon IV montrant la partie distale du corps central, la douille, la pince, le fourreau et le nez de guidage.
[Fig 5.] : vue agrandie selon V montrant la partie distale du corps central, la douille, la pince, le fourreau et le nez de guidage.
[Fig. 6] : vue en coupe longitudinale selon l'axe X d'un instrument selon l'invention, la pince étant en position fermée.
[Fig 7.] : vue agrandie selon VII montrant la partie distale du corps central, la douille, la pince, le fourreau et le nez de guidage lorsque la pince est en position fermée.

### DESCRIPTION DETAILLEE DE L'INVENTION

Dans le présent texte, les éléments qualifiés de « proximaux » sont les éléments les plus proches de la main du praticien. A l'opposé, les éléments qualifiés de « distaux » sont les éléments les plus éloignés de la main du praticien.

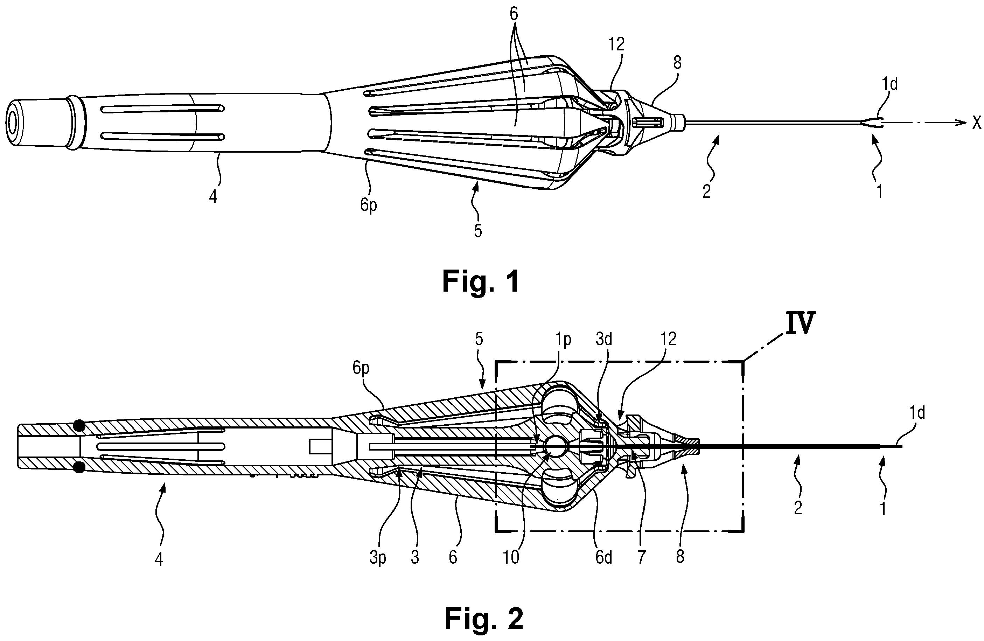

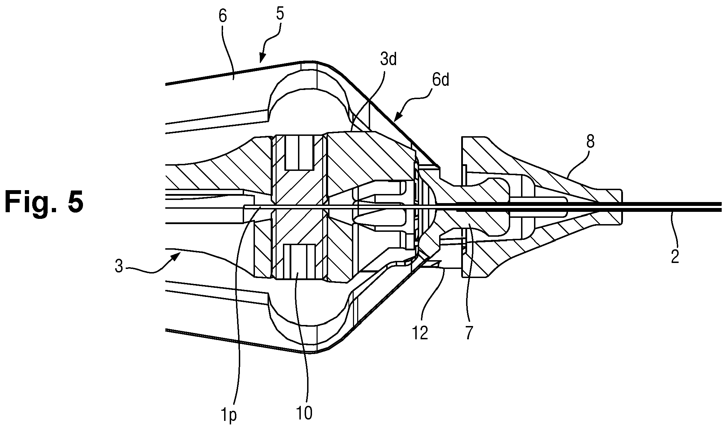

En référence aux figures 1 à 7 et selon un mode de réalisation particulier, la présente invention concerne un instrument, notamment une pince-rétine, comprenant une pince 1 en forme de tige avec une extrémité distale active 1d et une extrémité proximale 1p destinée à être fixée dans un corps central 3.

La pince 1 définit un axe longitudinal X de l'instrument passant par l'extrémité distale 1d de la pince l et l'extrémité proximale 1p de la pince 1.

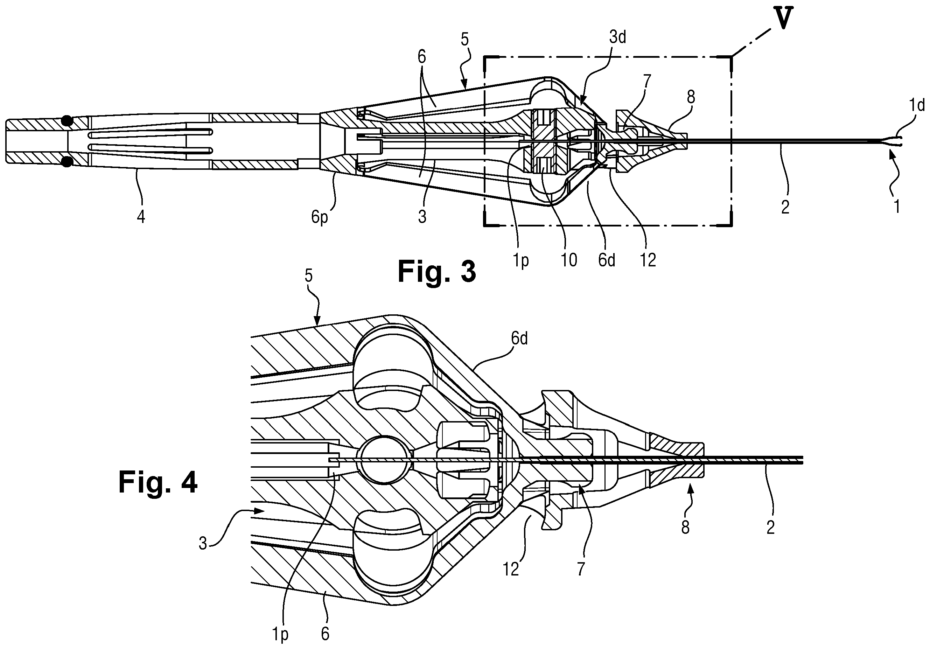

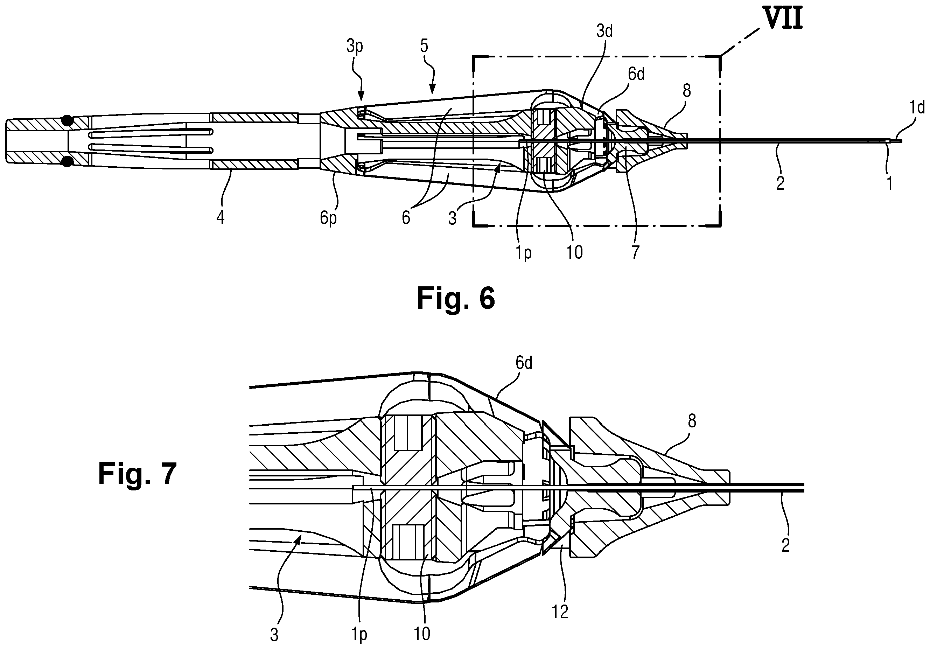

L'extrémité distale active 1d est séparée en deux branches élastiquement déformables terminées par une surface de préhension permettant de saisir un fragment tissulaire. En position désactivée, les deux branches de l'extrémité distale 1d de la pince 1 sont dans un état de repos dans lequel elles sont éloignées l'une de l'autre, comme cela est particulièrement illustré dans les figures 1 et 3. En position activée, les deux branches de l'extrémité distale 1d de la pince 1 sont dans un état de déformation élastique dans lequel elles sont en contact l'une avec l'autre de telle manière que les surfaces de préhension permettent la saisie du fragment tissulaire, comme cela est particulièrement illustré dans la figure 6. L'homme du métier saura choisir la pince 1 en fonction de l'utilisation finale de l'instrument objet de l'invention. L'instrument objet de l'invention comprend également un fourreau 2, en forme de tube cylindrique creux de diamètre interne légèrement supérieur à celui de la pince 1, dans laquelle la pince 1 est engagée à coulissement. Le diamètre est défini de telle sorte que la pince 1 une fois engagée dans le fourreau puisse coulisser à l'intérieur du fourreau entre une position activée et une position désactivée. En position activée, le fourreau 2 permet d'exercer un effort de resserrement sur les deux branches de l'extrémité distale 1d pour mettre en contact les surfaces de préhension des branches et ainsi fermer ou autrement dit activer la pince 1.

La pince 1 et le fourreau 2 sont réalisés en matériau biocompatible et stérilisable tel que, à titre d'exemples non limitatifs, en acier inoxydable ou en alliage chrome-cobalt.

L'instrument objet de l'invention comprend en outre un corps central 3, ici sensiblement en forme de tube creux. Le corps central 3 a une partie distale 3d, ici bombée, à laquelle est fixée l'extrémité proximale 1p. L'extrémité proximale 1p de la pince 1 est fixée dans la partie distale 3d du corps central 3 par au moins une vis 10. Ici, l'extrémité proximale 1p est fixée par deux vis 10 qui sont engagées transversalement à l'axe longitudinal X dans la partie distale 3d, en opposition l'une de l'autre, de manière à serrer entre elles l'extrémité proximale 1p. Ainsi, l'extrémité proximale 1p de la pince 1 est maintenue à l'intérieur du corps central 3 entre les deux vis 10 comme dans un étau. L'homme du métier peut ainsi ajuster le maintien de la pince 1, éventuellement la changer en serrant/desserrant l'une au moins des deux vis 10. L'homme du métier est à même de définir les dimensions du corps central 3 et les moyens de fixation de la pince 1 en fonction du matériau utilisé et des contraintes de fabrication. A titre d'illustration, ici le corps central est en Pa12 (Polyamide 12) d'un diamètre de 12 mm environ dans la partie la plus large du corps central et d'une longueur de l'ordre de 35 à 40 mm, de préférence 38 mm. Le corps central 3 a donc une extrémité proximale 3p, opposée à l'extrémité distale 3d, solidaire d'un manche 4 qui en forme le prolongement de sorte à faciliter la manipulation de l'instrument par la main du praticien. Le manche 4 est également de forme sensiblement cylindrique. L'homme du métier saura déterminer la longueur et le diamètre du manche en fonction de l'ergonomie souhaitée. A titre d'illustration, la longueur du manche est comprise entre 50 et 150 mm et le diamètre compris entre 7 et 10 mm.

L'instrument objet de l'invention comprend également un actionneur 5 formé d'un ensemble de leviers 6 s'étendant autour du corps central 3 et ayant des premières extrémités proximales 6p reliées au corps central 3 et des deuxièmes extrémités distales 6d qui s'étendent au-delà de la partie distale 3d et qui sont réunies par une douille 7 s'étendant en avant de la partie distale 3d et coaxialement à celle-ci. Le fourreau 2 est fixé à la douille 7 par tous moyens et par exemple par collage, soudage, ou simple maintien en force. Les leviers 6 sont élastiquement déformables entre une première position désactivée de la pince 1 dans laquelle l'extrémité active 1d de la pince 1 est ouverte et en saillie du fourreau 2 et une deuxième position activée dans laquelle le fourreau 2 vient recouvrir et resserrer l'extrémité active 1d de la pince 1 de manière à forcer la fermeture de l'extrémité active de la pince. Chaque levier 6 comprend un segment proximal et un segment distal qui sont reliés entre eux par une première lame flexible en définissant un angle ouvert inférieur à 180° lorsque l'actionneur 5 est en position désactivée, une deuxième lame flexible reliant le segment proximal au corps central 3 et une troisième lame flexible reliant le segment distal à la douille 7. Les segments et les lames flexibles sont en une seule pièce. En position activée, comme particulièrement illustré par la figure 6, les deux segments sont sensiblement alignés l'un avec l'autre, l'angle formé entre les deux segments étant alors sensiblement égal à 180°. L'homme du métier saura déterminer la longueur respective de chacun des segments de la lame ainsi que la valeur de l'angle formé en fonction de la longueur du corps central 3 et de la longueur de la course de la douille 7 pour que, en position activée, l'extrémité distale du fourreau 2 vienne fermer l'extrémité distale active 1d de la pince 1 sans recouvrir les surfaces de préhension de ladite extrémité distale 1d. Evidemment, l'homme du métier saura également prendre en compte la nature du matériau utilisé dans le dimensionnement des leviers et déterminer le nombre de leviers 6 nécessaires pour former l'actionneur 5 en fonction des dimensions de chaque levier, du matériau utilisé et de l'ergonomie souhaitée.

A titre d'illustration, l'actionneur 5 peut comprendre dix leviers 6 réalisés en Polyamide 12 (Pa12) ; chaque levier 6 a une largeur variable comprise de 1 à 5 mm pour le segment distal, et de 2 à 5 mm pour le segment proximal proche du manche 4. L'extrémité la plus fine des segments des leviers correspondant respectivement pour l'extrémité 6p à celle qui est à proximité de l'extrémité proximale 3p du corps et pour l'extrémité 6d à celle qui est dans le prolongement de la douille 7. La partie la plus large des segments se situant entre les deux et étant destinée à accueillir les doigts du praticien lorsqu'il utilise l'instrument. Le segment proximal et le segment distal de chaque levier 6 ont ici une longueur respective d'environ 30mm et inférieure à 10 mm et forment un angle d'environ 130° lorsque l'actionneur 5 est désactivé.

Les extrémités proximales 6p des leviers 6 sont ici d'une seule pièce avec la partie proximale 3p du corps central 3. A l'opposé, les extrémités distales 6d des leviers 6 sont d'une seule pièce avec la douille 7 qui est mobile en translation par rapport au corps central 3 le long de l'axe X. Ainsi, pour activer la pince, le manipulateur va exercer une pression sur tout ou partie des leviers 6 de l'actionneur 5, préférentiellement sur leur partie la plus large, les leviers 6 vont se déplier jusqu'à ce que les segments de chaque levier 6 forment entre eux un angle sensiblement égal à 180° en imprimant à la douille 7 et donc au fourreau 2 qui y est fixé un mouvement de translation le long de l'axe x en direction de l'extrémité distale 1d. L'extrémité distale du fourreau 2 va alors exercer une pression sur les deux branches de l'extrémité distale 1d de la pince 1 et provoquer le rapprochement de ces deux branches jusqu'à fermer ou autrement dit activer la pince 1. A l'inverse lorsque le praticien va relâcher la pression exercée sur l'actionneur 5, les leviers 6 vont se replier sous l'effet de leur élasticité jusqu'à ce que les deux segments forment entre eux l'angle initial. La douille 7 et le fourreau 2 vont réaliser une translation inverse vers le manche 4 : les deux branches de l'extrémité distale 1d de la pince 1 sont alors libérées de l'extrémité distale du fourreau 2 et s'écartent l'une de l'autre sous l'effet de leur élasticité. La course du fourreau 2 est arrêtée lorsque les leviers 6 sont dans leur état de repos et la douille 7 se trouve au voisinage de l'extrémité distale 3d du corps central 3.

Selon l'invention, le corps central 3, l'actionneur 5 et la douille 7 de l'instrument sont une pièce monobloc. L'ensemble corps central 3, actionneur 5 composé de l'ensemble de leviers 6 réunis par leur extrémité distale pour former la douille 7 sont réalisés par fabrication additive. Par exemple, le corps central, l'actionneur, et la douille peuvent être fabriqués en utilisant une imprimante de marque EOS alimenté avec du Polyamide 12 (Pa12), la fabrication se faisant par couches successives selon l'axe X.

Dans le mode de réalisation préféré de l'invention ici décrit, l'instrument selon l'invention comporte un nez de guidage 8, fixe et relié au corps central 3, qui s'étend en avant de la douille 7 et coiffe une partie de celle-ci. De manière préférée, le nez de guidage 8 est relié à l'extrémité distale du corps central 3 par des bras 12 passant entre les leviers 6. Les bras 12 sont ici dans le prolongement de la partie distale 3d du corps central 3. Le nez de guidage 8 comprend un perçage colinéaire à l'axe X qui reçoit à coulissement une partie du fourreau 2 de manière à guider ladite partie du fourreau 2 et limiter le risque d'une flexion du fourreau 2. Le diamètre du percement est légèrement supérieur au diamètre du fourreau 2 de sorte que ce dernier puisse coulisser librement dans le nez de guidage 8. Le nez de guidage 8 est ici sensiblement en forme de tronc de cône creux et percé en son sommet. Le nez de guidage 8 comprend en outre un logement arrière recevant librement une partie de la douille 7 notamment pour la position activée. Ainsi, en position activée de l'instrument, la douille 7 vient à proximité de la paroi interne du logement ménagé à l'intérieur du nez de guidage 8 tandis qu'en position désactivée de l'instrument, la douille 7 est voisine de l'extrémité distale du corps central 3. La course de la douille 7, et par conséquent du fourreau 2, se trouve donc comprise entre, d'une part, l'extrémité distale du corps central 3 et, d'autre part, la paroi interne du logement du nez de guidage 8. Il est rappelé que le nez de guidage est fixe. La présence d'un nez de guidage 8 permet d'améliorer la précision de l'instrument entre deux positions extrêmes. Il permet aussi de faciliter le coulissement du fourreau 2 le long de la pince 1 en en rigidifiant sa tenue améliorant ainsi la précision de l'instrument. En outre, le nez de guidage étant fixe et solidaire du corps central 3, la distance comprise entre l'extrémité 1d de la pince et le sommet du nez de guidage 8 reste constante, que l'instrument soit activé ou non. La longueur utile de l'ensemble pince 1 - fourreau 2 qui pénètre dans l'œil est ainsi totalement conservée. Ceci permet au praticien de manipuler l'instrument de la même façon que celui-ci soit activé ou non.

Conformément à l'invention, le nez de guidage 8, le corps central 3, l'actionneur 5 et la douille 7 sont fabriqués sous la forme d'une unique pièce monobloc. L'ensemble corps central 3, actionneur 5 composé de l'ensemble de leviers 6 réunis par leur extrémité distale pour former la douille 7, la douille 7 elle-même et le nez de guidage 8 dont les bras 12 passent entre les leviers 6 et sont dans le prolongement de la partie distale du corps central 3 est réalisé en fabrication additive.

De manière privilégiée, le manche 4 et le corps central 3 forme une pièce monobloc.

Ainsi, l'instrument selon l'invention comprend seulement trois éléments distincts :
- une pince 1
- un fourreau 2
- une seule et unique pièce monobloc comportant un corps central 3, un actionneur 5, une douille 7, et optionnellement un nez de guidage 8 et un manche 4.

Les coûts de fabrication s'en trouvent donc fortement réduits puisqu'il suffit de fabriquer la pièce monobloc par fabrication additive, puis de fixer le fourreau 2 dans la douille 7, enfin d'insérer à coulissement la pince 1 dans le fourreau 2 et la fixer dans le corps central 3, a fortiori lorsque la pièce monobloc comprend également le nez de guidage 8 et/ou le manche 4. L'invention concerne également un tel procédé de fabrication.

La fixation du fourreau 2 se fait de manière manuelle ou automatisée. Le fourreau 2 est inséré dans le percement du nez de guidage 8 puis dans le percement de la douille 7 où il est fixé. Enfin, une fois le fourreau 2 fixé dans la douille 7, la pince 1 est insérée à coulissement dans le fourreau 2 jusqu'à ce que son extrémité proximale 1p atteignent les moyens de fixation 10 liés au corps central 3. Une fois la pince 1 correctement positionnée, dans le corps central 3, les moyens de fixation, ici les vis 10 sont serrées de sorte à fixer la pince 1 et à en empêcher tout mouvement en translation selon l'axe X.

L'instrument conforme à l'invention comprend donc peu de pièces ce qui en simplifie l'assemblage et diminue les coûts de fabrication. L'utilisation de la fabrication additive permet de réaliser des formes difficiles voire impossibles à obtenir avec d'autres technologies : par exemple, cela permet de réaliser un instrument selon l'invention avec un nez de guidage 8 fixe solidaire du corps central 3.' Ce temps de fabrication, en particulier d'assemblage réduit et l'emploi de la fabrication additive permettent de produire des instruments facilement à un coût très faible tout en conservant l'ergonomie et la précision de l'instrument.

Bien entendu, l'invention n'est pas limitée au mode de réalisation décrit mais englobe toute variante entrant dans le champ de l'invention telle que définie par les revendications.

En particulier, la pince est maintenue dans le corps central par deux vis mais, l'extrémité proximale de la pince peut aussi être maintenue par une seule vis. L'extrémité proximale de la pince peut alternativement être collée ou soudée dans le corps central.

## Revendications

1. Instrument pour la chirurgie ophtalmique comprenant :
a. une pince (1) avec une extrémité active (1d) et un fourreau (2) recevant la pince (1) à coulissement
b. un corps central (3) avec une première extrémité proximale (3p) solidaire d'un manche (4) et une deuxième extrémité distale (3d) à laquelle est fixée une première extrémité de la pince (1p), opposée à l'extrémité active (1d) de ladite pince (1),
c. un actionneur(5) formé d'un ensemble de leviers (6) s'étendant autour du corps central (3) et ayant des premières extrémités (6p) reliées au corps central (3) et des deuxièmes extrémités (6d) réunies par une douille (7) à laquelle est fixée le fourreau (2), les leviers (6) étant élastiquement déformables entre une première position désactivée de la pince (1) dans laquelle l'extrémité active (1d) de la pince (1) est ouverte et en saillie du fourreau (2) et une deuxième position activée dans laquelle le fourreau (2) vient exercer un effort sur l'extrémité active (1d) de la pince (1) en forçant la fermeture de l'extrémité active (1d) de la pince (1)
dans lequel le corps central (3), l'actionneur (5) et la douille (7) sont une pièce monobloc, **caractérisé en ce que** ledit instrument comprend un nez de guidage (8) à travers lequel passe au moins une partie du fourreau (2) pour y être guidé, ledit nez de guidage étant fixe et relié au corps central (3).

2. Instrument selon la revendication 1 dans lequel le nez de guidage (8) est relié au corps central (3) par des bras passant entre les leviers (6).

3. Instrument selon les revendications 1 à 2 dans lequel le nez de guidage (8) comprend un logement recevant la douille (7) librement.

4. Instrument selon les revendications 1 à 3 dans lequel le nez de guidage (8), le corps central (3), l'actionneur (5) et la douille (7) sont une pièce monobloc.

5. Instrument selon l'une quelconque des revendications précédentes dans lequel le manche (4) et le corps central (3) forment une pièce monobloc.

6. Procédé de fabrication d'un instrument selon les revendications précédentes comprenant les étapes de :
a. fabriquer en fabrication additive une pièce monobloc comprenant au moins, un corps central (3), un actionneur (5), une douille (7) et un nez de guidage (8);
b. fixer le fourreau (2) dans la douille (7) ;
c. insérer à coulissement la pince dans le fourreau (2) et la fixer dans le corps central (3).

## Patentansprüche

1. Instrument für die Augenchirurgie, umfassend:
a. eine Pinzette (1) mit einem aktiven Ende (1d) und einer Hülse (2), in der die Pinzette (1) verschiebbar aufgenommen ist
b. ein Mittelteil (3) mit einem ersten proximalen Ende (3p), das fest mit einem Griff (4) verbunden ist, und einem zweiten distalen Ende (3d), an dem ein erstes Ende der Pinzette (1p) befestigt ist, das dem aktiven Ende (1d) dieser Pinzette (1) gegenüberliegt,
c. ein Betätigungselement (5), das aus einer Anordnung von Hebeln (6) besteht, die sich um das Mittelteil (3) herum erstrecken und erste Enden (6p) aufweisen, die mit dem Mittelteil (3) verbunden sind, sowie zweite Enden (6d), die durch eine Buchse (7) miteinander verbunden sind, an der die Hülse (2) befestigt ist, wobei die Hebel (6) elastisch verformbar sind zwischen einer ersten, deaktivierten Position der Pinzette (1), in der das aktive Ende (1d) der Pinzette (1) geöffnet ist und aus der Hülse (2) herausragt, und einer zweiten, aktivierten Position, in der die Hülse (2) eine Kraft auf das aktive Ende (1d) der Pinzette (1) ausübt und so das Schließen des aktiven Endes (1d) der Pinzette (1) erzwingt
wobei das Mittelteil (3), das Betätigungselement (5) und die Buchse (7) aus einem Stück bestehen, **dadurch gekennzeichnet, dass** das Instrument eine Führungsnase (8) umfasst, durch die mindestens ein Teil der Hülse (2) verläuft, um darin geführt zu werden, wobei die Führungsnase feststehend und mit dem Mittelteil (3) verbunden ist.

2. Instrument nach Anspruch 1, bei dem die Führungsnase (8) über Arme, die zwischen den Hebeln (6) verlaufen, mit dem Mittelteil (3) verbunden ist.

3. Instrument nach den Ansprüchen 1 bis 2, wobei die Führungsnase (8) eine Aufnahme umfasst, die die Buchse (7) frei aufnimmt.

4. Instrument nach den Ansprüchen 1 bis 3, wobei die Führungsnase (8), das Mittelteil (3), das Betätigungselement (5) und die Buchse (7) aus einem Stück bestehen.

5. Instrument nach einem der vorstehenden Ansprüche, bei dem der Griff (4) und das Mittelteil (3) aus einem Stück bestehen.

6. Verfahren zur Herstellung eines Instruments nach den vorstehenden Ansprüchen, das die folgenden Schritte umfasst:
a. Herstellung eines einteiligen Bauteils mittels additiver Fertigung, das mindestens ein Mittelteil (3), ein Betätigungselement (5), eine Buchse (7) und eine Führungsnase (8) umfasst;
b. Befestigung der Hülse (2) in der Buchse (7);
c. Einschieben der Pinzette in die Hülse (2) und Befestigung der Pinzette im Mittelteil (3).

## Claims

1. An instrument for ophthalmic surgery comprising:
a. a clamp (1) with an active end (1d) and a sleeve (2) receiving the sliding clamp (1)
b. a central body (3) with a first proximal end (3p) integral with a handle (4) and a second distal end (3d) to which is secured a first end of the clamp (1p), opposite the active end (1d) of said clamp (1),
c. an actuator (5) formed by a set of levers (6) extending around the central body (3) and having first ends (6p) connected to the central body (3) and second ends (6d) joined by a bushing (7) to which the sleeve (2) is secured, the levers (6) being elastically deformable between a first deactivated position of the clamp (1) in which the active end (1d) of the clamp (1) is open and protruding from the sleeve (2) and a second activated position in which the sleeve (2) exerts a force on the active end (1d) of the clamp (1) by forcing the closure of the active end (1d) of the clamp (1)
wherein the central body (3), the actuator (5) and the bushing (7) are a single piece, **characterized in that** said instrument comprises a guide nose (8) through which at least a part of the sleeve (2) passes to be guided therein, said guide nose being fixed and connected to the central body (3).

2. The instrument according to claim 1, wherein the guide nose (8) is connected to the central body (3) by arms passing between the levers (6).

3. The instrument according to claims 1 to 2 wherein the guide nose (8) comprises a housing receiving the bushing (7) freely.

4. The instrument according to claims 1 to 3 wherein the guide nose (8), the central body (3), the actuator (5) and the bushing (7) are a single piece.

5. The instrument according to any one of the preceding claims wherein the handle (4) and the central body (3) form a single piece.

6. A method of manufacturing an instrument according to the preceding claims comprising the steps of:
a. manufacturing by additive manufacturing a single piece comprising at least, a central body (3), an actuator (5), a bushing (7) and a guide nose (8);
b. securing the sleeve (2) in the bushing (7);
c. slidably inserting the clamp into the sleeve (2) and securing it in the central body (3).
